# EUROPEAN PATENT APPLICATION

(11) **EP 4 726 730 A1**
(43) Date of publication of application: **15.04.2026**
(21) Application number: 24821725.9
(22) Date of filing: 05.06.2024
(51) Int. Cl.: G16H 20/10, G06Q 30/015

(54) **METHOD FOR PROVIDING INFORMATION ON SHIPPING VOLUME OF MEDICINE**

(30) Priority: 12.06.2023 JP 2023095993
(71) Applicant: Cliniphar Ltd., Osaka-shi, Osaka, 540-0039 (JP)
(72) Inventor: TODA Kohei, Osaka-shi, Osaka 540-0039 (JP); MORIKAWA Saori, Osaka-shi, Osaka 540-0039 (JP)
(74) Representative: Peters, Sebastian Martinus
(86) International application number: PCT/JP2024/020536
(87) International publication number: WO 2024/257669

(57) **Abstract**

[Problem] To provide a method, system and programs to comprehensively identify and provide a user with information on the volume of shipment of pharmaceutical products.

[Solution] The method includes a shipment volume information collection step S110 in which a computer collects information on shipment volume of a plurality of pharmaceutical product s from a plurality of websites, a shipment volume registration step S120 in which the collected information on shipment volume of a plurality of pharmaceutical products is registered in a viewable database, and a shipment volume information step S130 which outputs the information on the shipment volume.

## Description

### TECHNICAL FIELD

This invention relates to a method for providing information regarding the shipment volume of pharmaceutical products.

### BACKGROUND ART

Japanese Patent Application Publication No. 2005-216239 discloses a pharmaceutical procurement support system. Japanese Patent Application Publication No. 2023-067395 discloses a pharmaceutical sales support device. Even with the use of these systems and devices, it is not possible to comprehensively grasp and provide information about the shipment volume of pharmaceutical products to users.

### CITATION LIST

### PATENT LITERATURE

Patent Literature 1: Japanese Patent Application Publication No. 2005-216239
Patent Literature 2: Japanese Patent Application Publication No. 2023-067395

### SUMMARY OF THE INVENTION

### TECHNICAL PROBLEM

An objective of this invention is to provide a method for comprehensively grasping and providing information about the shipment volume of pharmaceutical products to users.

### SOLUTIONS TO THE PROBLEM

This invention is fundamentally based on the insight that, by using specific terms related to shipment volume, information about the shipments volume of multiple pharmaceutical products can be collected in real-time and automatically by crawling relevant websites.

The first invention relates to a method for providing information on a shipment volume of pharmaceutical products by a computer. This method includes a shipment volume information collection step (S110) and a shipment volume registration step (S120). This method may further include a shipment volume information output step (S130) for outputting information related to the shipment volume. Additionally, the method may include a substitute pharmaceutical information provision step (S140). Furthermore, the method may include a manufacturer response status collection step (S111) and a manufacturer response status registration step (S121). Moreover, the method may also include a manufacturer response status output step (S131).

The shipment volume information collection step (S110) is a step in which a computer collects information regarding the shipment volume of multiple pharmaceutical products from multiple websites. The shipment volume information collection step (S110) may, for example, use terms such as "shipment status" or "shipment volume" included in multiple websites to collect information about the shipment volume of multiple pharmaceutical products.

The manufacturer response status collection step (S111) is a step in which a computer collects information regarding the response status of manufacturers from multiple websites.

The shipment volume registration step (S120) is a step in which the collected information regarding the shipment volume of multiple pharmaceutical products is registered in a database. The information registered in the database becomes viewable. The database can be searched using the pharmaceutical name, the pharmaceutical selling company name, or the pharmaceutical manufacturing company name.

The manufacturer response status registration step (S121) is a step in which the collected information regarding the manufacturer's response status is registered in the database.

The shipment volume information output step (S130) is a step for outputting information regarding the shipment volume.

The manufacturer response status output step (S131) is a step for outputting information regarding the response status of the manufacturer.

The substitute pharmaceutical information provision step (S140) is a step which provides information regarding pharmaceutical product which is a substitute for the pharmaceutical product targeted for search, and available for shipment, in a case the pharmaceutical product targeted for search is in a shipment adjustment state.

The second invention relates to the system 1 for providing information regarding the shipment volume of pharmaceutical products. This system is one which executes the method described above.

This system includes a shipment volume information collection unit 3, a shipment volume registration unit 5, and a shipment volume information output unit 7. The shipment volume information collection unit 3 is an element for collecting information regarding the shipment volume of multiple pharmaceutical products from multiple websites. The shipment volume registration unit 5 is an element for registering the collected shipment volume information of multiple pharmaceutical products into the database 9. The shipment volume information output unit 7 is an element for outputting the collected shipment volume information of multiple pharmaceutical products which have been registered in the database.

The third invention relates to a program or a non-temporary information recording medium on which the program is recorded for executing the method described above.

### ADVANTAGEOUS EFFECTS OF THE INVENTION

This invention provides a method for comprehensively grasping and providing information regarding the shipment volume of pharmaceutical products to users.

### BRIEF DESCRIPTION OF THE DRAWINGS

FIG. 1 is a flowchart for explaining an example of a method for providing information on the shipment volume of pharmaceutical products.
FIG. 2 is a block diagram for explaining an example of a system for providing information on the shipment volume of pharmaceutical products.
FIG. 3 is a conceptual diagram showing an example of the use of the system of the present invention.
FIG. 4 shows an example of a webpage related to the shipment of pharmaceutical products (AAAA tablet 100mg).
FIG. 5 shows an example of a webpage related to the shipment of other pharmaceutical products (BBBB tablet 50mg).
FIG. 6 is a conceptual diagram showing an example of the storage in a database.

### DESCRIPTION OF EMBODIMENTS

An embodiment for practicing the present invention will be described below with reference to the drawings. The present invention is not limited to the embodiment described below but also includes modifications that are made by those skilled in the art as appropriate within a scope obvious to those skilled in the art from the following embodiment.

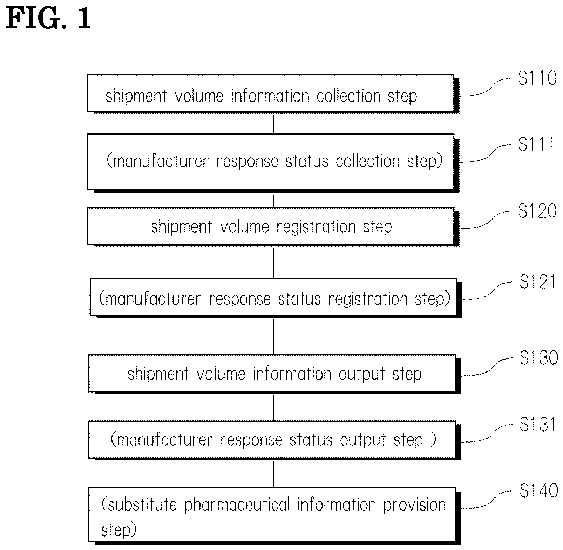

FIG. 1 is a flowchart for explaining an example of a method for providing information on the shipment volume of pharmaceutical products. As shown in FIG. 1, this method includes a shipment volume information collection step (S110) and a shipment volume registration step (S120). The method may further include a shipment volume information output step (S130) for outputting information on shipment volume. Additionally, this method may include a substitute pharmaceutical information provision step (S140). Furthermore, the method may include a manufacturer response status collection step (S111) and a response status registration step (S121). This method may also include a response status output step (S131).

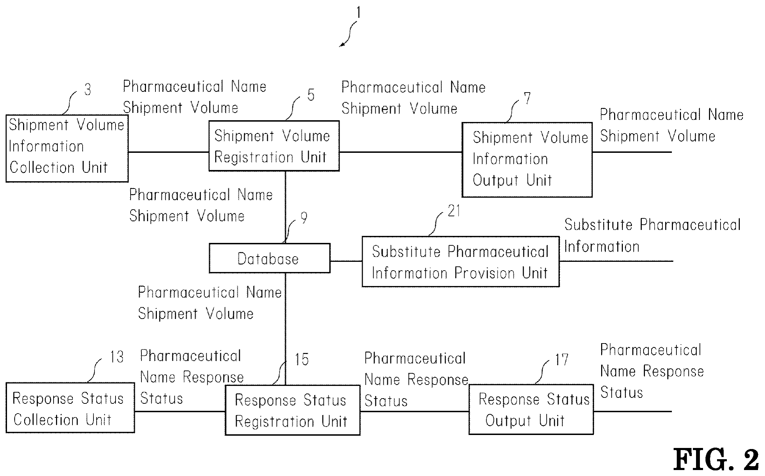

FIG. 2 is a block diagram for explaining an example of a system for providing information on the shipment volume of pharmaceutical products. As shown in FIG. 2, this system 1 includes a shipment volume information collection unit 3, a shipment volume registration unit 5, a shipment volume information output unit 7, and a database 9. The system 1 may further include a response status collection unit 13, a response status registration unit 15, and a response status output unit 17. Additionally, this system may include a substitute pharmaceutical information provision unit 21. This method and system are computer-based.

The computer includes an input unit, an output unit, a control unit, a processing unit, and a storage unit. Each component is connected via a bus or similar mechanism to facilitate the exchange of information. For example, the storage unit may store a control program or various types of information. When specific information is input through the input unit, the control unit reads the control program stored in the storage unit. The control unit then reads information stored in the storage unit and transfers the information to the computation unit as appropriate. The control unit also transfers input information to the computation unit as appropriate. The processing unit performs computational processing using the received information and stores the results in the storage unit. The control unit then retrieves the computation results stored in the storage unit and outputs them through the output unit. In this manner, various processes and steps are executed. The components and means responsible for executing these processes correspond to the various units. The computer may have a processor that implements the various functions and steps. It can operate as a standalone system or as part of a distributed system where functionality is divided between a server and terminals. In the latter case, it is preferable that the server and terminals are capable of exchanging information via networks such as the Internet or an intranet. The computer may include a processor and a memory connected to the processor. The memory may store instructions that, when executed by the processor, cause the computer to perform various steps or function as various components. The computer may also construct learning models by using training data and perform computations through machine learning. In such cases, the computer may execute various analyses and interpretations using learning models created through artificial intelligence (AI) techniques such as machine learning and deep learning.

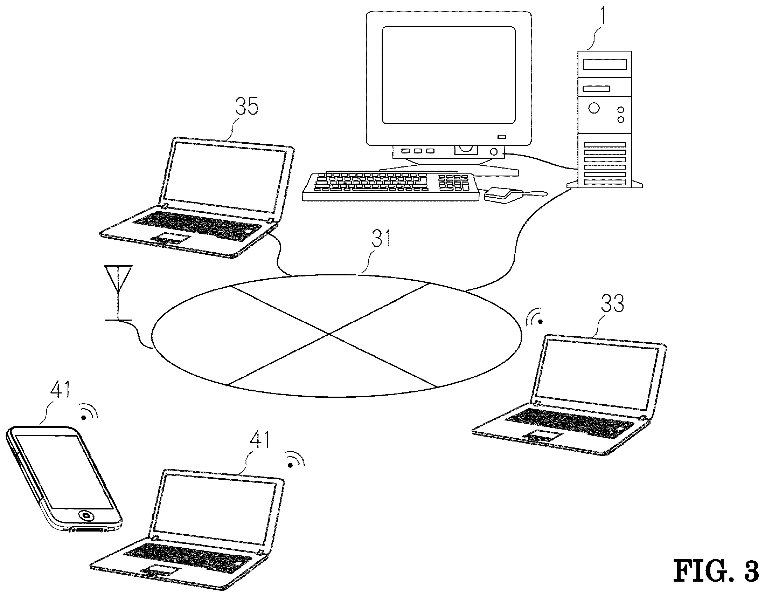

FIG. 3 is a conceptual diagram illustrating an example of using the system according to the present invention. As shown in FIG. 3, the system 1 for providing information on the shipment volume of pharmaceutical products (referred to simply as "system 1") is connected to multiple web servers 33, 35 via a network such as the Internet 31. Alternatively, the system 1 is configured to be able to access multiple web pages. The web servers, for example, are servers of pharmaceutical manufacturers which provide information regarding pharmaceutical products in a viewable state. As shown in FIG. 3, the system 1 is also connected to a user terminal 41. Thus, the user terminal 41 can display the information output by the system 1.

The following is a description of each step.

The shipment volume information collection step (S110) is a step in which the computer (system 1) collects information on the shipment volume of multiple pharmaceutical products from multiple websites. Preferably, the system 1 is configured to automatically crawl web pages containing pharmaceutical-related information, extract necessary information, and update the database. The pharmaceutical products may be pharmaceutical products for medical use or over-the-counter pharmaceutical products. The multiple websites are sites containing information on the shipment volume of pharmaceutical products or the response statuses of manufacturers and distributors for multiple pharmaceutical products. This website is typically associated with pharmaceutical company or manufacturer and distributor of pharmaceutical products. The shipment volume information collection unit 3 of the system 1 may store access information (e.g., URLs) for these websites in the storage unit. At any given timing (e.g., at regular intervals), the access information can be retrieved from the storage unit to access these websites. Additionally, the shipment volume information collection unit 3 may store a predetermined search keyword in the storage unit and retrieve the search keyword at any given timing to access specific websites. For specific website, the learning model used for access can be updated through machine learning. By doing so, the accuracy of the system 1 will be improved.

The shipment volume information collection unit 3 may collect information on the shipment volume of multiple pharmaceutical products by using terms related to "shipment status" or "shipment volume" included in multiple websites, or by using these terms. An example of information related to the shipment volume of multiple pharmaceutical products is the shipment volume of the manufacturer and distributor. The shipment volume information collection unit 3 extracts information on the shipment volume of multiple pharmaceutical products from the information contained in web pages related to pharmaceutical products. In this process, the shipment volume information collection unit 3 may also extract information related to the pharmaceutical products (pharmaceutical names) associated with the shipment volume of multiple pharmaceutical products.

Examples of pharmaceutical shipment volume are as follows:
A. Normal Shipment Volume: A shipment status that is generally 100% or more of the past or scheduled shipment volume.
B. Decreased Shipment Volume: A shipment status that is generally between 80% and 100% of the past or scheduled shipment volume.
C. Shipment Volume Impeded: A shipment status that is generally less than 80% of the past or scheduled shipment volume.
D. Shipment Suspension: A situation where shipments are not being made to the market.

Additionally, examples of information related to pharmaceutical shipment volume include: normal shipment volume, decreased shipment volume, shipment volume impeded, drastic shipment volume decrease, shipment difficulties, lifting of shipment suspension measures, discontinuation of sales, lifting of shipment suspension, and shipment suspension. These are merely examples, and other categorizations may also be used. Terms and synonyms that indicate pharmaceutical shipment volume may be stored in the storage unit as needed and may be used when extracting information or organizing the information.

The manufacturer and distributor response status collection step (S111) is a step in which a computer collects information on the response status of multiple pharmaceutical manufacturers and distributors from multiple websites. The response status collection unit 13 extracts information on the response status of the pharmaceutical manufacturer and distributor response status from web pages related to pharmaceutical products. For the sake of avoiding redundancy, the response status of the pharmaceutical manufacturer and distributor will simply be referred to as "response status."

Examples of manufacturer and distributor response status are as follows:
1. Normal Shipment: A situation where all orders can be fulfilled, or sufficient inventory is secured.
2. Limited Shipment (Due to Company Reasons): A situation where, due to the company's circumstances, not all orders can be fulfilled.
3. Limited Shipment (Due to the Impact of Other Companies' Products): A situation where, due to the impact of other companies' products, not all orders can be fulfilled.
4. Limited Shipment (Other Reasons): A situation where, due to other reasons, not all orders can be fulfilled.

These are merely examples, and other categorizations may also be used. Terms and synonyms indicating the manufacturer and distributor response status may be stored in the storage unit as needed and used when extracting or organizing information.

The shipment volume registration step (S120) is a step in which information on the shipment volume of multiple pharmaceutical products collected is registered in the database 9. The information registered in the database 9 (some of the information) is made available for viewing. It is preferable for the database 9 to be searchable using pharmaceutical names, pharmaceutical sales company names, or pharmaceutical manufacturing company names. The database 9 may store, for example, the pharmaceutical name, pharmaceutical sales company name, pharmaceutical manufacturing company name, information on the shipment volume of pharmaceutical shipment volume, information on the manufacturer and distributor's response status, information on substitute pharmaceutical products, information on the types of substitute pharmaceutical products, pharmaceutical identification information, pharmaceutical prices, and information on the pharmaceutical's selling price, all related to the pharmaceutical name. The database 9 may store some of these types of information. The shipment volume registration unit 5 stores (or updates) the extracted information on the pharmaceutical's shipment volume in a searchable format in the database 9. This allows searching for the shipment volume and response status of a target pharmaceutical product using the pharmaceutical name, pharmaceutical sales company name, or pharmaceutical manufacturing company name.

The response status registration step (S121) is a step in which information on the response status of the manufacturer and distributor collected is registered in database. The response status registration unit 15 stores the extracted information on the pharmaceutical manufacturer and distributor response status in the database 9, or updates the information stored in the database 9 using the information on the pharmaceutical manufacturer and distributor response status. This operation is performed for multiple websites (web pages).

The shipment volume information output step (S130) is a step for outputting information related to shipment volume. The shipment volume information output unit 7 outputs the information stored in the database in response to a request from the user (or the user's terminal 41), for example.

The response status output process (S131) is a step for outputting information related to the response status of the manufacturer and distributor. The response status output unit 17 outputs information regarding the pharmaceutical manufacturer and distributor response status in response to a request from the user (or the user's terminal 41).

Preferably the system 1 automatically (for example, periodically) crawls relevant websites and updates the database. This allows the system 1 to provide real-time and automatic information on the shipment volume of multiple pharmaceutical products.

The substitute pharmaceutical information provision step (S140) is a step that provides information regarding pharmaceutical products which are substitutes for the pharmaceutical products targeted for search and available for shipment in a case the pharmaceutical products targeted for search is in a shipment adjustment state. The shipment adjustment status refers to a situation where pharmaceutical products shipment is difficult due to shipment volume or response status. The system 1 may determine whether pharmaceutical products are in shipment adjustment status based on either or both the shipment volume and response status. To do this, information regarding shipment adjustment status may be stored in the memory based on each status, allowing the system 1 to determine shipment adjustment status based on pharmaceutical products shipment volume or response status. The database 9 stores information regarding substitute pharmaceutical products for each drug (1. Pharmaceutical products with the same active ingredient for the same disease, 2. Pharmaceutical products with a different active ingredient for the same disease, 3. Pharmaceutical products with the same active ingredient for a different disease). For example, if a user (on terminal 41) inquires about the situation of a pharmaceutical product A, system 1 will check the shipment volume of pharmaceutical product A in the database 9. If the shipment volume is "suspended," system 1 will retrieve information about alternative pharmaceutical product B (such as shipment volume or response status). If pharmaceutical B's shipment volume is "normal," system 1 will output information about pharmaceutical B (such as pharmaceutical name, package insert, whether it is available for supply, manufacturer, active ingredient, and sales data) to the user (on terminal 41), in addition to the information about pharmaceutical A.

The program described in this specification causes a computer to perform the above processes and function as the system 1. Such a program is stored, for example, in memory. The program enables the processor or computer to execute the above steps. Examples of storage media include CD, CD-ROM, DVD, USB memory, hard disk, and disk on a server.

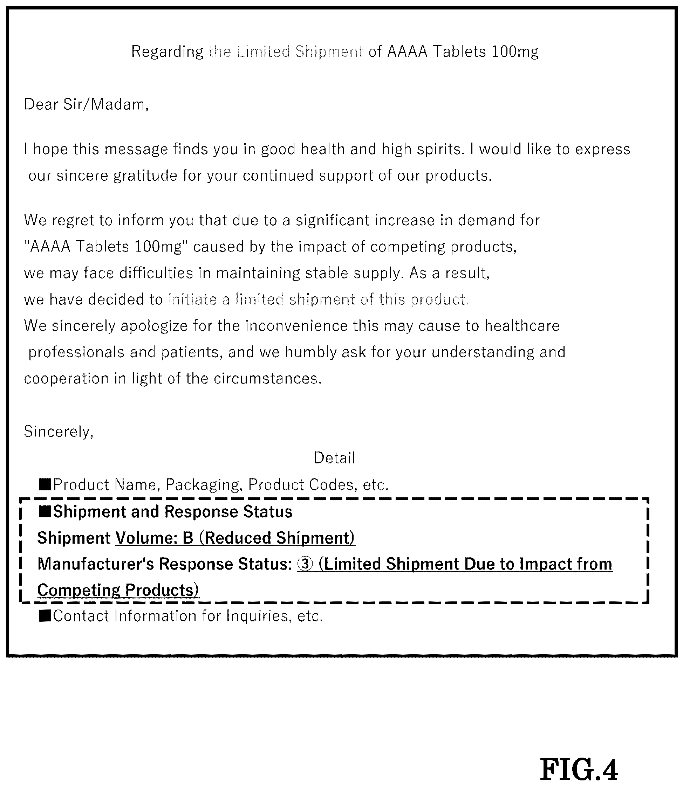

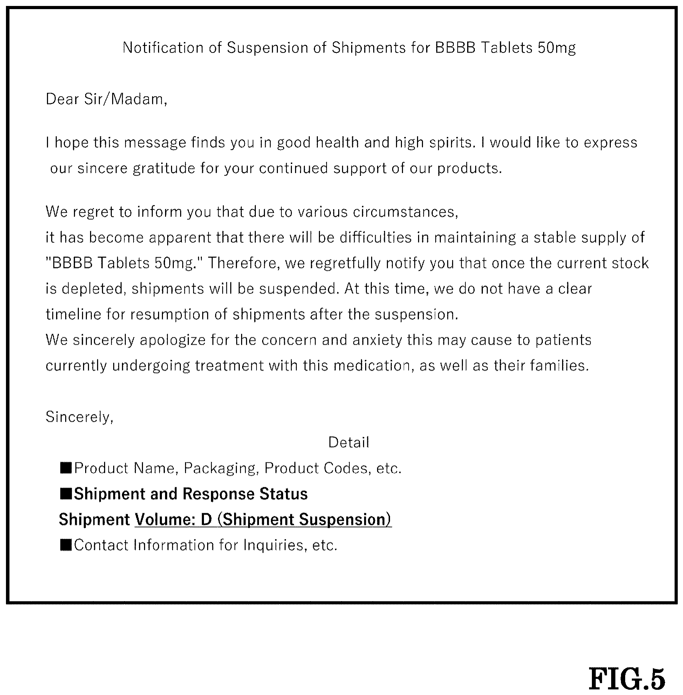

FIG. 4 is an example of a web page concerning the shipment of a pharmaceutical product (AAAA tablet 100mg). FIG. 5 is an example of a web page concerning the shipment of another pharmaceutical product (BBBB tablet 50mg). FIG. 4 and FIG. 5 are cited from "Definitions of Terms Related to the Supply Status of Pharmaceutical products and Future Information Provision" by the Japan Pharmaceutical Manufacturers Association Stability Assurance Committee, dated May 31, 2022.

The shipment volume information collection unit 3 extracts the pharmaceutical name from the section which states "AAAA tablet 100mg" included in the web page shown in FIG. 4. Then, the shipment volume registration unit 5 registers (or updates) the extracted pharmaceutical name in the database 9. The shipment volume information collection unit 3 also extracts shipment volume information for "AAAA tablet 100mg" from the section stating "Shipment volume: B, 'Reduced shipment'" on the web page in FIG. 4. The shipment volume registration unit 5 records (or updates) the extracted pharmaceutical shipment volume information as "Shipment volume: B, 'Reduced shipment'" in the database 9. Since the expression and locations of shipment volume information can vary by webpage, the learning model may be updated using the various patterns as teacher data. This improves the accuracy of collecting shipment volume information in the system 1.

The shipment volume information collection unit 3 extracts the pharmaceutical name "BBBB tablet 50mg" From the web page in FIG. 5 and extracts shipment volume information ("Shipment suspended") from the corresponding section. The shipment volume registration unit 5 records (or updates) the extracted shipment volume information of the pharmaceutical in the database 9. Additionally, the shipment volume information collection unit 3 can analyse text from the web page, such as the sentence "this will affect stable supply," to obtain shipment volume information about pharmaceutical "BBBB tablet 50mg." This approach of analysing text on a webpage to gather information about pharmaceutical shipment volume is one implementation of the system 1.

In the example in FIG. 4, the response status collection unit 13 extracts the pharmaceutical name ("AAAA tablet 100mg") and, in relation to the phrase "response status of the pharmaceutical manufacturer", extracts the response status information ("Limited shipment (due to other pharmaceutical impact)"). The response status registration unit 15 records this extracted response status information in database 9, or using this information updates the information related to the response status of pharmaceutical "AAAA tablet 100mg."

In the example in FIG. 5, the response status collection unit 13 extracts the pharmaceutical name ("BBBB tablet 50mg"), extracts the phrase "shipment suspended" from the webpage, classifies the response status of the pharmaceutical manufacturer using a learning model and obtains the response status information (e.g., "Limited shipment (other reasons)"). The response status registration unit 15 records (or updates) the extracted response status information in the database 9 or updates the information on the response status of the manufacturer and distributor of the pharmaceutical (BBBB tablets 50 mg) using this information.

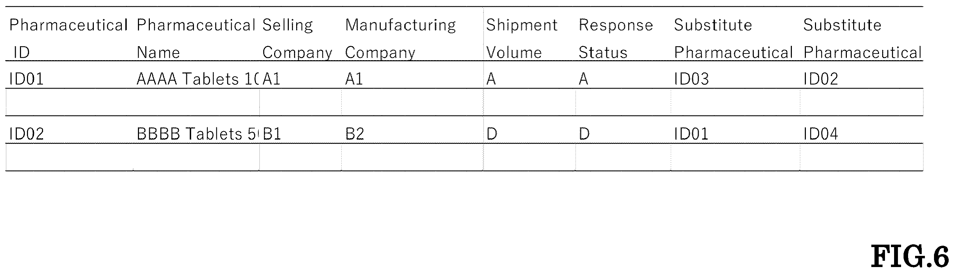

FIG. 6 shows a conceptual diagram of an example of data stored in the database. In the example shown in FIG. 6, information about pharmaceutical name, manufacturer and distributor name, shipment volume, response status, substitute pharmaceutical, and related information are stored in connection with the pharmaceutical name.

For example, if a user (on terminal 41) accesses a website provided by the system 1 through the internet 31 and enters the pharmaceutical name "BBBB tablet 50mg" in the search section of the website, the system 1 will retrieve the shipment volume information for "BBBB tablet 50mg" from database 9 (e.g., "Shipment suspended"). The system 1 displays this information on the website. Additionally, the system 1 will search for a substitute pharmaceutical product for "BBBB tablet 50mg" stored in database 9. For instance, a pharmaceutical product with the same active ingredient (e.g., "AAAA tablet 100mg," a generic of BBBB tablet) is retrieved. The system 1 also retrieves shipment volume and response status information for pharmaceutical "AAAA tablet 100mg." Then, the system1 outputs information so that the user (on terminal 41) can display information about "AAAA tablet 100mg" which is substitute for the pharmaceutical product and available through the website.

### INDUSTRIAL APPLICABILITY

This invention relates to the sale of pharmaceutical products and can be used in e-commerce or pharmaceutical sales businesses. Additionally, the program and information recording media of this invention can be effectively used by companies providing e-commerce services.

### REFERENCE SIGNS LIST

1 system for providing information regarding the shipment volume of pharmaceutical products
3 shipment volume information collection unit
5 shipment volume registration unit
7 shipment volume information output unit
9 database
13 response status collection unit
15 response status registration unit
17 response status output unit
21 substitute pharmaceutical information provision unit
31 Internet
33 web server
35 web server
41 user terminal

## Claims

1. A method for providing information related to shipment volume of pharmaceutical products, the method comprising:
by a computer,
a shipment volume information collection step of reading a predetermined search keyword from a memory, with the read search keyword, accessing multiple websites, crawling the multiple websites, and collecting information regarding the shipment volume of multiple pharmaceutical products; and
a shipment volume registration step of registering the collected information regarding the shipment volume of multiple pharmaceutical products in a database wherein the information registered in the database is made available for viewing, wherein
the method further comprises a substitute pharmaceutical information provision step of providing information regarding a pharmaceutical substitute which is a substitute for the pharmaceutical product targeted for search and available for shipment, in a case a pharmaceutical product targeted for search is in a shipment adjustment state, and
the pharmaceutical substitute for the pharmaceutical product targeted for search is selected from
a pharmaceutical product for the same disease as the disease for which the pharmaceutical product targeted for search is, and the active ingredient of which is the same as the active ingredient of the pharmaceutical product targeted for search,
a pharmaceutical product for the same disease as the disease for which the pharmaceutical product targeted for search is, and the active ingredient of which is different from the active ingredient of the pharmaceutical product targeted for search, and
a pharmaceutical product for the disease different from the disease for which the pharmaceutical product targeted for search is , but the active ingredient of which is the same as the active ingredient of the pharmaceutical product targeted for search.

2. The method for providing the information related to the shipment volume of the pharmaceutical products according to claim 1, wherein the shipment volume information collection step is a step of collecting information on the shipment volume of multiple pharmaceutical products by using terms "shipment status" or "shipment volume" included in the multiple websites.

3. The computer method for providing the information related to the shipment volume of the pharmaceutical products according to claim 1, wherein the database is searchable using pharmaceutical name, the name of a pharmaceutical sales company, or the name of a pharmaceutical manufacturing company.

4. The computer method for providing the information related to the shipment volume of the pharmaceutical products according to claim 1, further comprising:
a manufacturer response status collection step of collecting information on a response status of the pharmaceutical manufacturer from the multiple websites; and
a response status registration step of registering the collected information on the response status of pharmaceutical manufacturer into the database.

5. A program for causing a computer to execute a method for providing information related to shipment volume of pharmaceutical products, the method comprising:
a shipment volume information collection step of reading a predetermined search keyword from a memory, with the read search keyword, accesses multiple websites using the read search keyword, crawls the multiple websites, and collects information regarding the shipment volume of multiple pharmaceutical products; and
a shipment volume registration step of registering collected information regarding the shipment volume of multiple pharmaceutical products in a database and making the information registered in the database viewable, wherein
the method further comprises a substitute pharmaceutical information provision step of providing information regarding a pharmaceutical substitute which is a substitute for the pharmaceutical product targeted for search and available for shipment, in a case a pharmaceutical product targeted for search is in a shipment adjustment state, and
the pharmaceutical substitute for the pharmaceutical product targeted for search is selected from a pharmaceutical product for the same disease as the disease for which the pharmaceutical product targeted for search is, and the active ingredient of which is the same as the active ingredient of the pharmaceutical product targeted for search,
a pharmaceutical product for the same disease as the disease for which the pharmaceutical product targeted for search is, and the active ingredient of which is different from the active ingredient of the pharmaceutical product targeted for search, and
a pharmaceutical product for the disease different from the disease for which the pharmaceutical product targeted for search is , but the active ingredient of which is the same as the active ingredient of the pharmaceutical product targeted for search.

6. A non-transitory computer readable information recording medium storing the program according to claim 5.

7. A method for providing information related to shipment volume of pharmaceutical products, the method comprising:
by a computer,
a shipment volume information collection step of reading access information to multiple websites, accessing multiple websites, crawling the multiple websites, and collecting information regarding the shipment volume of multiple pharmaceutical products; and
a shipment volume registration step of registering collected information regarding the shipment volume of multiple pharmaceutical products in a database, and making the information registered in the database viewable, wherein
the method further comprises a substitute pharmaceutical information provision step of providing information regarding a pharmaceutical substitute which is a substitute for a pharmaceutical product targeted for search and available for shipment, in a case the pharmaceutical product targeted for search is in a shipment adjustment state, and
the pharmaceutical substitute for the pharmaceutical product targeted for search is selected from
a pharmaceutical product for the same disease as the disease for which the pharmaceutical product targeted for search is, and the active ingredient of which is the same as the active ingredient of the pharmaceutical product targeted for search,
a pharmaceutical product for the same disease as the disease for which the pharmaceutical product targeted for search is, and the active ingredient of which is different from the active ingredient of the pharmaceutical product targeted for search, and
a pharmaceutical product for the disease different from the disease for which the pharmaceutical product targeted for search is, but the active ingredient of which is the same as the active ingredient of the pharmaceutical product targeted for search.
